# EUROPÄISCHE PATENTANMELDUNG

(11) **EP 2 216 012 A2**
(43) Veröffentlichungstag der Anmeldung: **11.08.2010**
(21) Anmeldenummer: 09015186.1
(22) Anmeldetag: 08.12.2009
(51) Int. Cl.: A61K 8/34, A61K 8/49, A61Q 19/06

(54) **Wärmende kosmetische oder dermatologische Zubereitung**

(30) Priorität: 04.02.2009 DE 102009007528
(71) Anmelder: Beiersdorf AG, 20245 Hamburg (DE)
(72) Erfinder: Ratschow, Cécile, Dr., 22761 Hamburg (DE); Eckert, Julia, 22085 Hamburg (DE); Kröpke, Rainer, 22869 Schenefeld (DE); Fänger, Sabine, 22763 Hamburg (DE)
(74) Vertreter: Wilke, Jochen

(57) **Zusammenfassung**

Kosmetische oder dermatologische Wiricstoffkombinationen umfassend Vanillylbutylether und eine oder mehrere durchblutungsfördernde Substanzen.

## Beschreibung

Die vorliegende Erfindung betrifft wärmend wirksame Wirkstoffkombinationen sowie wärmende kosmetische oder dermatologische Zubereitungen, solche Wirkstoffkombinationen enthaltend.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommenden Stoffen. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Aufgabe der kosmetischen Hautpflege ist es, die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) zu stärken oder wiederherzustellen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Übliche kosmetische Zubereitungen liegen in Form von Emulsionen vor. Emulsionen selbst wirken beim Verteilen auf der Haut nicht wärmend. Herkömmliche Hautpflegeprodukte haben vielmehr eher eine kühlende Wirkung auf die Haut. Diese entsteht dadurch, dass das in den Produkten enthaltene Wasser auf der Haut verdunstet und bei diesem Prozess der Haut Wärme entzieht. Wenn die Haut abkühlt schließen sich auch ihre Poren und die Durchblutung der feinen Blutkapillaren im papillären Comium welche die Haut (auch die gefäßlose Epidermis) versorgen, wird reduziert. Dieser Umstand führt dazu, dass die in den Hautpflegeprodukten enthaltenen Wirkstoffe nur ungenügend von der Haut aufgenommen werden.

Bekannt ist auch, unerwünschte Hauterscheinungen wie beispielsweise Cellulite durch Wärmeanwendung zu bekämpfen.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Zwar gibt es auch bisher schon eine Reihe von Salben und Pflastern (z.B. "ABC-Pflaster"), welche auf der Haut ein Wärmegefühl erzeugen, doch werden diese Effekte durch eine chemische Reizung der Haut (z.B. mit Bienengift) erzeugt.

Es war daher die Aufgabe der vorliegenden Erfindung kosmetische und/oder dermatologische Zubereitungen zu entwickeln, die einen wärmenden Effekt auf die Haut haben, ohne diese chemisch zu reizen.

Es war nun die Aufgabe der vorliegenden Erfindung, selbstwärmende kosmetische Zubereitungen zu entwickeln, die intensiveres Wärmegefühl hervorrufen, sich angenehmer auf der Haut anfühlen und besser auf der Haut zu verteilen sind.

Überraschenderweise wird die Aufgabe gelöst durch kosmetische oder dermatologische Wirkstoffkombinationen umfassend Vanillylbutylether und eine oder mehrere durchblutungsfördernde Substanzen.

Ebenfalls Gegenstand der vorliegenden Erfindung sind kosmetische oder dermatologische Zubereitungen, enthaltend Vanillylbutylether und eine oder mehrere durchblutungsfördernde Substanzen.

Vanillylbutylether ist durch folgende chemische Struktur gekennzeichnet und wird von der Gesellschaft Takasago unter der Warenbezeichnung "Hotact VBE" vertrieben.

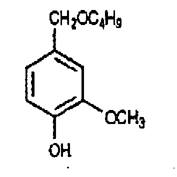

Die erfindungsgemäßen Zubereitungen zeichnen sich durch eine gute kosmetische Eigenschaften auf der Haut aus.

Bevorzugte durchblutungsfördernde Substanz ist das Methylnicotinat, welches sich durch folgende Struktur auszeichnet:

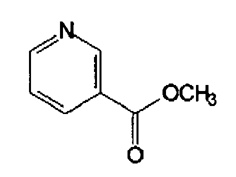

Vorteilhaft wird das Gewichtsverhältnis aus Vanillylbutylether einerseits und der Gesamtmenge einer oder mehrerer durchbtutungsfördernder Substanzen andererseits aus dem Bereich von 100 : 1 bis 1 zu 100, bevorzugt 10 : 1 bis 1 zu 10, insbesondere bevorzugt 2 : 1 bis 1 : 2 gewählt.

Kosmetische oder dermatologische Zubereitungen gemäß der Erfindung enthalten vorteilhaft 0,0001 bis 25 Gew.-%, bevorzugt 0.01 bis 10 Gew.-%, insbesondere bevorzugt 0,05 bis 5 Gew.-% an Vanillylbutylether, bezogen auf das Gesamtgewicht der Zubereitungen.

Kosmetische oder dermatologische Zubereitungen gemäß der Erfindung enthalten vorteilhaft 0,0001 bis 10 Gew.-%, bevorzugt 0,001 bis 5 Gew.-%, insbesondere bevorzugt 0,005 bis 1 Gew.-% an einer oder mehreren durchblutungsfördernden Substanzen, insbesondere Methylnicotinat, bezogen auf das Gesamtgewicht der Zubereitungen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Eine besonders bevorzugte Ausführungsform der vorliegenden Erfindung besteht in der Verwendung erfindungsgemäßer Wirkstoffkombinationen zur Herstellung kosmetischer oder dermatologischer Zubereitungen gegen Cellulite.

Die kosmetischen und dermatologischen Zubereitungen gemäß der Erfindung können kosmetische oder galenische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feucht haltende Substanzen, Fette, Öle, Wachse oder andere übliche Bestandteile einer kosmetischen oder dermatofogischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Zubereitungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

| **Beispiele** | **1** | **2** | **3** | **4** | **5** |
|---|---|---|---|---|---|
| | **Gew.-%** | | | | |
| Glycerylstearatcitrat | 3,0 | --- | --- | --- | --- |
| Polyethylenglykol(40)stearat | --- | 5 | --- | --- | --- |
| Polyglyceryl-2 Dipolyhydroxystearat | --- | --- | 4 | --- | --- |
| PEG/PPG-18/18 Dimethicon | --- | --- | --- | 2 | --- |
| Myristylmyristat | 1 | 5,5 | --- | --- | --- |
| Sorbitanstearat | --- | 1,5 | 3 | --- | --- |
| Zyklisches Silikonöl | --- | 1,5 | --- | 20 | 5 |
| Lineares Silikonöl | 0,5 | 2 | 1 | --- | --- |
| Acrylates/C10-30 Alkyl Acrylate Crosspolymer | --- | --- | --- | --- | 0,2 |
| Chondrus Crispus | --- | --- | --- | --- | 0,4 |
| Natriumpolyacrylat | --- | --- | --- | --- | 0,15 |
| Natriumcarbomer | 0,5 | 1 | --- | --- | 2 |
| Ethanol | 2 | 5 | --- | 10 | 8 |
| Cetylstearylalkohol | 3 | --- | --- | --- | --- |
| Medizinisches Weissöl | 1 | --- | 5 | --- | --- |
| Dicaprylylcarbonat | 2,5 | 3 | --- | --- | 1 |
| Natriumchlorid | --- | --- | --- | 1,5 | --- |
| Octyldodecanol | 5 | 1,0 | 0,75 | --- | --- |
| Parfum | q,s, | q,s, | q,s, | q,s, | q,s, |
| Methylparaben | 0,4 | 0,2 | 0,3 | 0,05 | --- |
| Phenoxyethanol | 0,1 | --- | 0,3 | 0,5 | 0,5 |
| Propylparaben | --- | 0,4 | 0,25 | --- | --- |
| Vanillylbutylether | 0,5 | 2 | 0,3 | 0,01 | 5 |
| Methylnicotinat | 0,2 | 0,05 | --- | 3 | 0,001 |
| Butylenglykol | --- | --- | 4 | 10 | --- |
| Glycerin | 5 | 10 | 3 | 20 | 7,5 |
| C13-16 Isoparaffin | --- | 4 | 2 | --- | 1 |
| Wasser | ad 100 | ad 100 | ad 100 | ad 100 | ad 100 |

| | |
|---|---|
| **Wirknachweis der synergistischen Wirkung der beiden Substanzen sowie der hautverträglichen Wirkung von Hotact VBE auf Methylnicotinat:** | |
| Home-in-Use-Test: | |
| 44 Probandinnen testeten 3 Produkte (O/W-Grundlage mit verschiedenen Wirkstoffkombinationen, siehe unten). Jedes Produkt wurde 5 Tage angewendet, zwischen 2 Produkte wurde am Wochenende pausiert. | |
| | Produkt 1 enthielt 0,6% Hotact VBE |
| | Produkt 2 enthielt 0,05% Methylnicotinat |
| | Produkt 3 enthielt 0,6% Hotact VBE und 0,05% Methylnicotinat |
| | |
| Ergebnisse für den Wirknachweis der synergistischen Wirkung der beiden Substanzen: | |
| | 89% der Probandinnen haben mit Produkt 1 eine Wärme gespürt |
| | 84% der Probandinnen haben mit Produkt 2 eine Wärme gespürt |
| | 95% der Probandinnen haben mit Produkt 3 eine Wärme gespürt |
| → Durch die Kombination beider Wirksteffe empfinden mehr Probandinnen eine Wärme als durch die Wirkstoffe einzeln. | |
| Ergebnisse für den Wirknachweis der hautverträglichen Wirkung von Hotact VBE auf Methylnlcotinat:: | |
| 38% der Probandinnen bekommen mit Produkt 2 eine feurige Rötung. | |
| Mit Produkt 3 bekommen nur noch 27% der Probandinnen eine feurige Rötung → Durch Hotact VBE wird Methylnicotinat hautverträglicher. | |

**Im Test verwendete Rezepturen**

| inci | Produkt 1 | Produkt 2 | Produkt 3 |
|---|---|---|---|
| Ethanol | 3 | 3 | 3 |
| Wasser | 72,8265 | 73,3765 | 72,7765 |
| Wasser + Natriumhydroxide | 0,08 | 0,08 | 0,08 |
| Caprylic/Capric Triglyceride | 4 | 4 | 4 |
| Carbomer | 0,2 | 0,2 | 0,2 |
| Cetearylalcohol | 1,8 | 1,8 | 1,8 |
| Dimethicone | 2 | 2 | 2 |
| Glycerin | 8,6935 | 8,6935 | 8,6935 |
| Glycerylstearatcitrate | 1,8 | 1,8 | 1,8 |
| methylnicotinat | | 0,05 | 0,05 |
| Methylparaben | 0,3 | 0,3 | 0,3 |
| Myristylmyristat | 3 | 3 | 3 |
| Octyldodecanol | 1 | 1 | 1 |
| Parfum | 0,3 | 0,3 | 0,3 |
| Phenoxyethanol | 0,4 | 0,4 | 0,4 |
| Vanillylbutylether | 0,6 | | 0,6 |

## Patentansprüche

1. Kosmetische oder dermatologische Wirkstoffkombinationen umfassend Vanillylbutylether und eine oder mehrere durchblutungsfördernde Substanzen.

2. Kosmetische oder dermatologische Zubereitungen, enthaltend Wirkstoffkombinationen nach Anspruch 1.

3. Wirkstoffkombinationen nach Anspruch 1 oder Zubereitungen nach Anspruch 2, **dadurch gekennzeichnet, daß** als durchblutungsfördernde Substanz Methylnicotinat gewählt wird.

4. Wirkstoffkombinationen nach Anspruch 1 oder Zubereitungen nach Anspruch 2, **dadurch gekennzeichnet, daß** das Gewichtsverhältnis aus Vanillylbutylether einerseits und der Gesamtmenge einer oder mehrerer durchblutungsfördernder Substanzen andererseits aus dem Bereich von 100 : 1 bis 1 zu 100, bevorzugt 10 : 1 bis 1 zu 10, insbesondere bevorzugt 2 : 1 bis 1 : 2 gewählt wird.

5. Zubereitungen nach einem oder mehreren der vorstehenden Ansprüche, **dadurch gekennzeichnet, daß** sie 0,0001 bis 25 Gew.-%, bevorzugt 0,01 bis 10 Gew.-%, insbesondere bevorzugt 0,05 bis 5 Gew.-% an Vanillylbutylether enthalten, bezogen auf das Gesamtgewicht der Zubereitungen.

6. Zubereitungen nach einem oder mehreren der vorstehenden Ansprüche, **dadurch gekennzeichnet, daß** sie 0,0001 bis 10 Gew.-%, bevorzugt 0,001 bis 5 Gew.-%, insbesondere bevorzugt 0,005 bis 1 Gew.-% an einer oder mehreren durchblutungsfördernden Substanzen, insbesondere Methylnicotinat enthalten, bezogen auf das Gesamtgewicht der Zubereitungen.

7. Zubereitungen nach einem oder mehreren der vorstehenden Ansprüche, **dadurch gekennzeichnet, daß** sie nach Applikation auf der Haut die Hauttemperatur um mindestens 0,6 °C erhöhen.

8. Verwendung von Wirkstoffkombinationen nach einem der vorstehenden Ansprüche zur Herstellung kosmetischer oder dermatologischer Zubereitungen gegen Cellulite.

9. Verwendung von Wirkstoffkombinationen nach einem der vorstehenden Ansprüche zur Herstellung kosmetischer oder dermatologischer Zubereitungen als Figurpflege für eine schöner wirkende Silhouette, gestrafft und remodeliert.

10. Verwendung von Wirkstoffkombinationen nach einem der vorstehenden Ansprüche zur Herstellung kosmetischer oder dermatologischer Zubereitungen für spürbares Wohlbefinden und gepflegtes Hautgefühl.
